# EUROPÄISCHE PATENTSCHRIFT

(11) **EP 2 242 851 B1**
(45) Veröffentlichungstag und Bekanntmachung des Hinweises auf die Patenterteilung: **09.11.2011**
(21) Anmeldenummer: 08855754.1
(22) Anmeldetag: 02.12.2008
(51) Int. Cl.: C12Q 1/68

(54) **VERFAHREN ZUR DIAGNOSE EINER GENETISCHEN PRÄDISPOSITION FÜR EINE GEFÄSSERKRANKUNG**
METHOD FOR DIAGNOSING A GENETIC PREDISPOSITION FOR VASCULAR DISEASE
PROCÉDÉ POUR DIAGNOSTIQUER UNE PRÉDISPOSITION GÉNÉTIQUE À UNE MALADIE VASCULAIRE

(30) Priorität: 03.12.2007 DE 102007058340
(43) Veröffentlichungstag der Anmeldung: 27.10.2010
(73) Patentinhaber: Johann Wolfgang Goethe-Universität, 60325 Frankfurt am Main (DE)
(72) Erfinder: TEGEDER, Irmgard, 60598 Frankfurt (DE); LÖTSCH, Jörn, 60316 Frankfurt am Main (DE); CHANNON, Keith, M., Oxford OX3 6TH (GB)
(74) Vertreter: Krauss, Jan
(86) Internationale Anmeldenummer: PCT/DE2008/001990
(87) Internationale Veröffentlichungsnummer: WO 2009/071058

(56) Entgegenhaltungen:
- WO-A-2008/028687
- US-A1- 2007 254 288
- LOTSCH J ET AL: "Reliable screening for a pain-protective haplotype in the GTP cyclohydrolase1 gene (GCH1) through the use of 3 or fewer single nucleotide polymorphisms" CLINICAL CHEMISTRY, AMERICAN ASSOCIATION FOR CLINICAL CHEMISTRY, WASHINGTON, DC, Bd. 53, Nr. 6, 1. Januar 2007 (2007-01-01), Seiten 1010-1015, XP009093143 ISSN: 0009-9147
- ZHANG LIAN ET AL: "Discovery of common human genetic variants of GTP cyclohydrolase 1 (GCH1) governing nitric oxide, autonomic activity, and cardiovascular risk." THE JOURNAL OF CLINICAL INVESTIGATION SEP 2007, Bd. 117, Nr. 9, September 2007 (2007-09), Seiten 2658-2671, XP002523599 ISSN: 0021-9738
- ANTONIADES C ET AL: "GCH1 Haplotype Determines Vascular and Plasma Biopterin Availability in Coronary Artery Disease" JOURNAL OF THE AMERICAN COLLEGE OF CARDIOLOGY, ELSEVIER, NEW YORK, NY, US, Bd. 52, Nr. 2, 8. Juli 2008 (2008-07-08), Seiten 158-165, XP022797630 ISSN: 0735-1097 [gefunden am 2008-07-01]
- RAO F ET AL: "Complex Trait Genetics" JOURNAL OF THE AMERICAN COLLEGE OF CARDIOLOGY, ELSEVIER, NEW YORK, NY, US, Bd. 52, Nr. 2, 8. Juli 2008 (2008-07-08), Seiten 166-168, XP022797631 ISSN: 0735-1097 [gefunden am 2008-07-01]
- LÖTSCH JÖRN ET AL: "Reliable screening for a pain-protective haplotype in the GTP cyclohydrolase 1 gene (GCH1) through the use of 3 or fewer single nucleotide polymorphisms.", CLINICAL CHEMISTRY JUN 2007 LNKD- PUBMED:17363416, [Online] vol. 53, no. 6, 15 March 2007 (2007-03-15) , pages 1010-1015, ISSN: 0009-9147 Supplemental Data Retrieved from the Internet: URL:http://www.clinchem.org/cgi/content/fu ll/clinchem.2006.082883/DC1> [retrieved on 2010-12-03]

### SEQUENCE LISTING

<110> Johann Wolfgang Goethe-Universität Frankfurt am Main
<120> Verfahren zur Diagnose einer genetischen Prädisposition für eine Gefäßerkrankung
<130> U60024PCT
<150> DE 102007058340.2
   <151> 03.12.2007
<160> 9
<170> PatentIn version 3.3
<210> 1
   <211> 18
   <212> DNA
   <213> Artificial
<220>
   <223> Primer
<400> 1
   tggggtgagg gttgagtt 18
<210> 2
   <211> 21
   <212> DNA
   <213> Artificial
<220>
   <223> Primer
<220>
   <221> biotin
   <222> (1) .. (1)
<400> 2
   aatgttaaca caataggagc g 21
<210> 3
   <211> 23
   <212> DNA
   <213> Artificial
<220>
   <223> Primer
<400> 3
   gctatttgct ttgtccacct cta 23
<210> 4
   <211> 24
   <212> DNA
   <213> Artificial
<220>
   <223> Primer
<220>
   <221> biotin
   <222> (1) .. (1)
<400> 4
   aacctggaac tgagaattgt tcac 24
<210> 5
   <211> 24
   <212> DNA
   <213> Artificial
<220>
   <223> Primer
<400> 5
   atcctttcaa tctggaactg actg 24
<210> 6
   <211> 24
   <212> DNA
   <213> Artificial
<220>
   <223> Primer
<220>
   <221> biotin
   <222> (1) .. (1)
<400> 6
   gcattctaaa atcagggaaa atca 24
<210> 7
   <211> 20
   <212> DNA
   <213> Artificial
<220>
   <223> Sequencing primer
<400> 7
   cttgaatgac tgaagtttgg 20
<210> 8
   <211> 16
   <212> DNA
   <213> Artificial
<220>
   <223> Sequencing primer
<400> 8
   cccacctgac tcattt 16
<210> 9
   <211> 20
   <212> DNA
   <213> Artificial
<220>
   <223> Sequencing primer
<400> 9
   ggtgtgtgta tgtacaactt 20

## Beschreibung

Die Erfindung betrifft ein Verfahren zur Diagnose einer genetischen Prädisposition für eine Gefäßerkrankung, insbesondere für eine koronare Arterienerkrankung (CAD), und seine Verwendung zur Diagnose einer genetischen Prädisposition für eine Gefäßerkrankung in einem Individuum. Weiterhin betrifft die Erfindung einen Kit zur Diagnose einer genetischen Prädisposition für eine Gefäßerkrankung und die Verwendung eines derartigen Kits.

Der Erhalt der endothelialen Funktion stellt einen kritischen Aspekt der vaskulären Homöostase dar. Der Verlust der normalen endothelialen Produktion von Stickoxid (NO) stellt ein frühes und charakteristisches Merkmal vaskulärer Erkrankungen dar und spielt wahrscheinlich eine Rolle in der Pathogenese derartiger Erkrankungen. Die endotheliale Stickoxidsynthase (eNOS) wird durch den Co-Faktor Tetrahydrobiopterin (BH4) reguliert (1 bis 3). Eine reduzierte Verfügbarkeit von BH4 als Folge einer Erkrankung scheint einen wichtigen Aspekt der beobachteten beeinträchtigten eNOS-Aktivität und der erhöhten vaskulären Superoxidproduktion in Tiermodellen darzustellen (1, 3). In Menschen mit vaskulären Erkrankungen kann die akute Gabe von BH4 die endotheliale Funktion verbessern (4, 5) und Behandlungen mit Folaten oder Vitamin C, die die endotheliale Funktion verbessern, vermitteln möglicherweise zumindest Teile ihrer Wirksamkeit durch Erhöhung der BH4-Verfügbarkeit (6 bis 8). Trotz allem ist es bisher unklar geblieben, in wie fern Veränderungen der endogenen BH4-Verfügbarkeit eine direkte Rolle in der Modulation endothelialer Funktion in Menschen *in vi*vo spielen könnten.

Die GTP-Cyclohydrolase I (GTPCH) ist das geschwindigkeitsbestimmende Enzym bei der Biosynthese von BH4 (9, 19) und stellt durch transkriptionale Regulation der *GCH1* Expression sowie posttranslationale Modifikation eine der wichtigsten Determinanten der BH4 Konzentrationen dar. In einigen experimentellen Modellen waren reduzierte BH4-Konzentrationen mit reduzierter GTPCH-Aktivität assoziiert (11). Es ist jedoch auch möglich, dass BH4 gegenüber der Oxidation durch freie Radikale wie Peroxinitrit (ONOO⁻) empfindlich ist, wodurch Dihydrobiopterin (BH2) und Biopterin (B) entsteht, ohne dass es zu einer signifikanten Veränderung in der Konzentration der Gesamt-Biopterine kommt (6, 8). Die relative Bedeutung der Synthese von BH4 gegenüber der Oxidation von BH4 im Rahmen der Arteriosklerose ist komplex, da eine lokale und symmetrische Entzündung die Hochregulation der *GCH1* Expression zu Folge hat (12), aber auch die ONOO⁻-Produktion erhöht, was möglicherweise zu einer größeren BH4-Oxidation führt (13).

US 2007/254288 beschreibt eine Methode zur Diagnose des Risikos eines Patienten, BH4-assoziierte Erkrankungen zu entwickeln, indem die allelischen Varianten des GCH1-Gens anhand von 182 SNPs bestimmt werden. Wie auf S. 14, [0068] beschrieben, wurden von den 182 SNPs 15 ausgewählt, die "gleichmäßig über das Gen verteilt" sind, und zur Definition eines schmerzunempfindlichen Haplotyps herangezogen (Fig. 11A). Die in der US 2007/254288 offenbarten Beispiele und Daten zeigen allein einen Zusammenhang zwischen den beschriebenen SNPs und Schmerzempfindlichkeit

In der WO 2008/028687, die als Stand der Technik gemäß Artikel 54(3) EPÜ zitiert wird, wird die Verwendung eines Kits in einem Diagnoseverfahren zur Diagnose einer genetischen Prädisposition für eine Gefäßerkrankung gemäß der vorliegenden Erfindung nicht offenbart. Lötsch et al. (in Lötsch J, et al. Reliable screening for a pain-protective haplotype in the GTP cyclohydrolase 1 gene (GCH1) through the use of 3 or fewer single nucleotide polymorphisms. Clin Chem. 2007 Jun;53(6):1010-5. Epub 2007 Mar 15) beschreiben die im neuen Anspruch 1 genannten drei SNPs im Zusammenhang mit einem schmerzunempfindlichen Haplotyp.

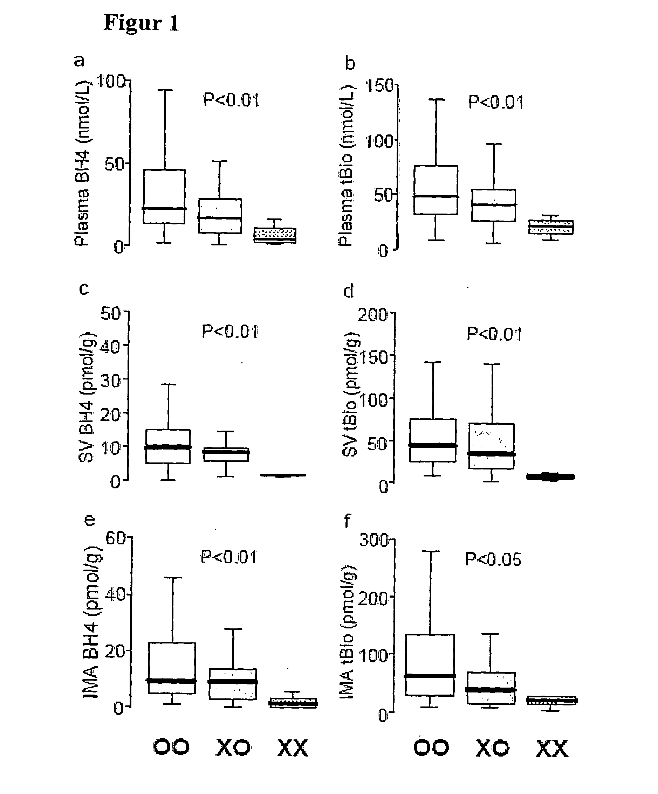

Zhang et al. (in Zhang L, et al. Discovery of common human genetic variants of GTP cyclohydrolase 1 (GCH1) governing nitric oxide, autonomic activity, and cardiovascular risk. J Clin Invest. 2007 Set; 117(9):2658-71) beschreibt die Durchführung und die Ergebnisse einer umfangreichen Studie zur Korrelation von genetischen Varianten von GCH1 mit kardiovaskulärem Risiko. Wie in Figur 1 von Zhang et al. dargestellt, liegen der Studie umfangreiche Sequenzierungsarbeiten des gesamten GCH1 Genes zu Grunde. Trotz des großen Umfangs der Studie konnte lediglich ein SNP, das als "C + 243 T" bezeichnet wird, im 3-untranslatierten Bereichs des GCH1 Gens identifiziert werden, dass lediglich eine schwache Assoziation mit einem erhöhten Risiko fiir einen Hypertonus bei gesunden Trägern dieses SNP zeigt.

### Beschreibung der Erfindung

Um bei der Diagnose von Gefäßerkrankungen nicht auf die schwierige Interpretation von Konzentrationen von intrazellulären Metaboliten angewiesen zu sein, deren Regulation in der Zelle noch weitgehend unbekannt ist, wäre eine Diagnose aufgrund genetischer Parameter wünschenswert. Demgemäß lag der vorliegenden Erfindung die Aufgabe zu Grunde, die Prädisposition für eine Gefäßerkrankung in einfacher und leicht interpretierbarer Weise zu ermöglichen.

Das der Erfindung zugrunde liegende Problem wird durch ein Verfahren zur Diagnose einer genetischen Prädisposition für eine Gefäßerkrankung, insbesondere einer koronaren Arterienerkrankung (CAD) gelöst. Erfindungsgemäß umfasst das Verfahren die folgenden Schritte:
- Zunächst wird eine Probe bereitgestellt, die eine von dem genomische Lokus des Gens GCH1 abgeleitete Nukleinsäure enthält. Unter dem Begriff "abgeleitete Nukleinsäure" wird sowohl die genomische DNS, als auch eine unprozessierte, also nicht-gespleisste RNS (auch als hnRNS bezeichnet) und/oder eine davon abgeleitete cDNS verstanden.
- In einem zweiten Schritt des erfindungsgemäßen Verfahrens wird die Anwesenheit oder Abwesenheit eines Nukleotidpolymorphismus (SNP) des Gens *GCH1* in der Nukleinsäure bestimmt. Der Nukleotidpolymorphismus, der einen spezifischen GCH1 Haplotyp beschreibt, ist dabei ausgewählt aus der Gruppe bestehend aus rs8007267G>A, rs3783641A>T und rs10483639C>G. Die Anwesenheit mindestens eines dieser SNPs zeigt eine genetische Prädisposition für eine Gefäßerkrankung an. In einer bevorzugten Ausführungsform des erfindungsgemäßen Verfahrens wird die An- oder Abwesenheit von mindestens zwei, besonders bevorzugt von allen drei genannten Nukleotidpolymorphismen bestimmt.

Die Nomenklatur der genannten SNPs basiert auf der dbSNP-Zugangsnummer, die dem Fachmann bekannt ist.

Neben der Bestimmung der Anwesenheit oder Abwesenheit mindestens eines der oben genannten Nukleotidpolymorphismen des Gens *GCH1* in der Nukleinsäure kann auch die Bestimmung der Anwesenheit oder Abwesenheit eines SNPs, das als Block mit dem Haploblock aufweisend die oben genannten Nukleotidpolymorphismen vererbt wird, bestimmt werden.

Der Nukleotidpolymorphismus rs8007267G>A ist im 5'-untranslatierten Bereich des Gens *GCH1* zu finden, während der Nukleotidpolymorphismus rs3783641A>T im Intron 1 und der Nukleotidpolymorphismus rs10483639C>G im 3'-untranslatierten Bereich des *GCH1* Gens zu finden ist. Aufgrund dieses Umstands kann das erfindungsgemäße Verfahren nur mit solchen Nukleinsäuren durchgeführt werden, die die genannten im untranslatierten Bereich des Gens angeordneten Nukleotidpolymorphismen abbilden.

Mittels des erfindungsgemäßen Verfahrens können Individuen identifiziert werden, die eine genetische Prädisposition für eine Gefäßerkrankung, insbesondere für koronare Arterienerkrankung aufweisen. Die genannten Nukleotidpolymorphismen schlagen sich dabei in den betroffenen Individuen in den folgenden pathobiochemischen Parameter nieder: Zum einen zeigen diese Individuen eine reduzierte *GCH1* Expression in den Gefäßwänden. Weiterhin ist eine reduzierte Konzentration von Biopterinen, insbesondere von BH4 im Blutplasma und in den Blutgefäßwänden feststellbar. Schließlich weisen die betroffenen Individuen eine veränderte Regulation der endothelialen Stickoxidsynthase (eNOS) in den Zellen der Blutgefäße auf. Letzteres führt zu einer verminderten Konzentration von Stickoxid (NO) im Blutplasma und in den Gefäßwänden, zu einer erhöhten vaskulären Superoxidproduktion, sowie einer Reduktion der Acetylcholin-vermittelten Vasorelaxation, wobei letzteres auf eine endotheliale Dysfunktion hinweist. Die verwendeten Begriffe "reduziert", "erniedrigt", "vermindert" und "erhöht" beziehen sich jeweils auf eine statistisch signifikante Abweichung gegenüber einem gesunden Individuum, das die genannten Nukleotidpolymorphismen nicht aufweist.

In einer bevorzugten Ausführungsform des Verfahrens stammt die Probe von einem Säugetier, insbesondere von einem Menschen.

In einer bevorzugten Ausführungsform des erfindungsgemäßen Verfahrens wird der Nachweis der Anwesenheit oder Abwesenheit eines Nukleotidpolymorphismus des Gens *GCH1* in der Nukleinsäure durch Sequenzierung, Primerverlängerung, Hybridisierung, Restriktionsfragment-Analyse, Oligonukleotidligation und/oder eine allel-spezifische PCR erbracht. In den meisten Anwendungsfällen wird es vorteilhaft sein, eine Nachweismethode zu wählen, mittels derer eine Hochdurchsatzgenotypisierung durchgeführt werden kann. Dazu stehen grundsätzlich vier verschiedene Mechanismen bereit, nämlich die allel-spezifische Primerverlängerung, die allel-spezifische Hybridisierung, die allel-spezifische Oligonukleotidligation und die allel-spezifische Spaltung einer "flap probe" (42).

Die Sequenzierung bzw. die Primerverlängerungstechnologie basiert auf der Bestimmung der Sequenz an einer bestimmten Basenposition, wobei die Verlängerung eines Primers an dieser bestimmten Stelle nur dann erfolgt, wenn eine bestimmte Base im Templat vorliegt (Landegren et al., Genome Res. 8, 769 bis 776 (1998)). Allel-spezifische Hybridisierungsprotokolle basieren auf der Erkennung eines oder mehrerer SNPs. Dazu wurden verschiedene Techniken entwickelt, die beispielsweise in den folgenden Dokumenten beschrieben sind (Livak, Genet Anal. 14, 143 (1999); Tyagi et al., Nat. Biotechnol. 16, 49(1998)).

Die Hybridisierung wird für die Genotypisierung auch im Zusammenhang mit so genannten Mikrochips verwendet, die in verschiedenen Publikationen beschrieben sind (Hacea et al., Nature Genetics 14, 441 (1996); Shoemaker et al., Nature Genetics 14, 450 (1996); Chee et al., Sciences 274, 610 (1996); DeRisi et al., Nature Genetics 14, 457 (1996); Fun et al., Genome Res. 10, 853 (2000)).

Allel-spezifische Oligonukleotidligationsassays haben den Vorteil einer hohen Spezifität, insbesondere, da diese Verfahren beispielsweise mit der Fluoreszenz-Resonanz-Energie-Transfer (FRET) -Technologie verwendet werden können (Chen et al., Genome Res. 8, 549 (1998)).

Das Vorhandensein eines der genannten Polymorphismen kann grundsätzlich mit jeder Körperzelle ermittelt werden. Besonders vorteilhaft ist es, wenn die bereitgestellte Probe aus einer Körperflüssigkeit gewonnen wird, da diese im Allgemeinen leicht, unter Anwendung nur minimal invasiver Methoden gewinnbar ist. Geeignete Körperflüssigkeiten schließen Blut, Ejakulat, Speichel, bronchiale Lavage, Pleuraeffusion, Peritonealflüssigkeit, Amnionflüssigkeit oder Mundschleimhautabstrich ein. In einer alternativen Ausführungsform des erfindungsgemäßen Verfahrens wird die bereitgestellte Probe aus einem Gewebe, etwa einem Blutgefäß gewonnen.

Das der Erfindung zugrunde liegende Problem wird ebenfalls durch die Verwendung eines Kit zur Diagnose einer genetischen Prädisposition für eine Gefäßerkrankung, insbesondere für eine koronare Arterienerkrankung gelöst. Erfindungsgemäß weist ein derartiger Kit mindestens eine Sonde und/oder mindestens einen Primer zum Nachweis mindestens eines Nukleotidpolymorphismus des Gens *GCH1* auf, wobei der Nukleotidpolymorphismus ausgewählt ist aus der Gruppe bestehend aus rs8007267G>A, rs3783641A>T und rs10483639C>G.

In einer bevorzugten Ausführungsform ist der Primer des Kits ein Amplifikationsprimer oder eine Sequenzierungsprimer, so dass der jeweilige Nukleotidpolymorphismus mittels Amplifikation, beispielsweise im Rahmen einer Polymerase Kettenreaktion oder mittels einer Sequenzierung bestimmt werden kann. Die Sonde oder der Primer können mit einer geeigneten Markierung markiert sein, so dass der Kit beispielsweise die Durchführung einer Echtzeit-PCR erlaubt. In einer alternativen Ausführungsform weist der Kit eine Sonde aus, die an einer festen Basis angeordnet ist, so dass ein Biochip vorliegt.

Die bevorzugten Primer zur PCR Amplifikation der erfindungsgemäßen *GCH1* Gensegmente und zur Sequenzierung sind im Folgenden angegeben.

PCR Primer für:
- dbSNP rs8007267 G>A:
   vorwärts: 5'-TGGGGTGAGGGTTGAGTT-3' (SEQ ID No. 1),
   revers: 5'-biotin-AATGTTAACACAATAGGAGCG-3' (SEQ ID No. 2);
- dbSNP rs3783641 A>T:
   vorwärts: 5'-GCTATTTGCTTTGTCCACCTCTA-3' (SEQ ID No. 3),
   revers: 5-biotin-AACCTGGAACTGAGAATTGTTCAC-3' (SEQ ID No. 4);
- dbSNP rs10483639 C>G:
   vorwärts: 5'-ATCCTTTCAATCTGGAACTGACTG-3' (SEQ ID No. 5),
   revers: 5'-biotin-GCATTCTAAAATCAGGGAAAATCA-3' (SEQ ID No. 6).

Sequenzierungsprimer für:
- dbSNP rs8007267 G>A:
   5'-CTTGAATGACTGAAGTTTGG-3' (SEQ ID No. 7);
- dbSNP rs3783641 A>T:
   5'-CCCACCTGACTCATTT-3' (SEQ ID No. 8);
- dbSNP rs10483639 C>G:
   5'-GGTGTGTGTATGTACAACTT-3' (SEQ ID No. 9).

Das der Erfindung zugrunde liegende Problem wird ebenfalls durch die Verwendung eines oben beschrieben Verfahrens zur Diagnose einer genetischen Prädisposition für eine Gefäßerkrankung, insbesondere für eine koronare Arterienerkrankung (CAD) in einem Individuum gelöst. Neben der Verwendung des genannten Verfahrens zur Diagnose kann das Verfahren auch beispielsweise in der Forschung angewandt werden.

Die Erfindung wird nachfolgend anhand von Beispielen näher erläutert, ohne auf diese Beispiele beschränkt zu sein. Die Ergebnisse der in den Beispielen beschriebenen Versuche sind in den Figuren dargestellt.

### Figuren

Es zeigen:
Figur 1: Das Vorhandensein des seltenen Haplotyps (XO oder XX) war mit signifikant niedrigeren Konzentrationen von Plasma Tetrahydrobiopterin (BH4, a) und Gesamt-Biopterin (tBio, b) assoziiert. Ein ähnlicher Effekt konnte auch in Saphenusvenen (SV, c und d) und internen Arterien der Mamma (IMA, e und f) beobachtet werden.

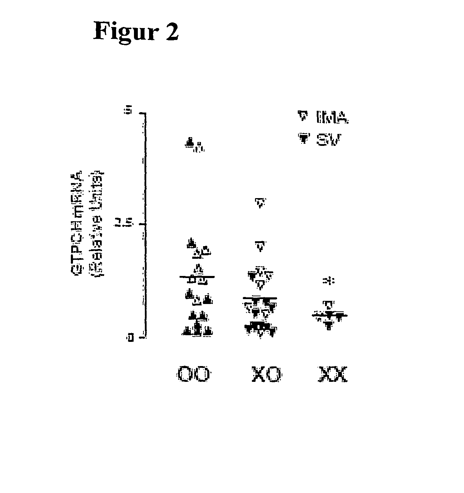

Figur 2: Das Auftreten des XX-Genotyps (homozygot für den GCH1-Polymorphismus) im Vergleich zum OO-Genotyp (homozygot Wildtyp CGH1) war mit signifikant niedrigeren mRNA-Konzentrationen von GTPCH assoziiert. Es sind Ergebnisse aus Versuchen gezeigt, die mit 23 Saphenusvenen (SV) und 17 internen Arterien der Mamma (IMA) erhalten wurden. **P* < 0,05 vs OO.

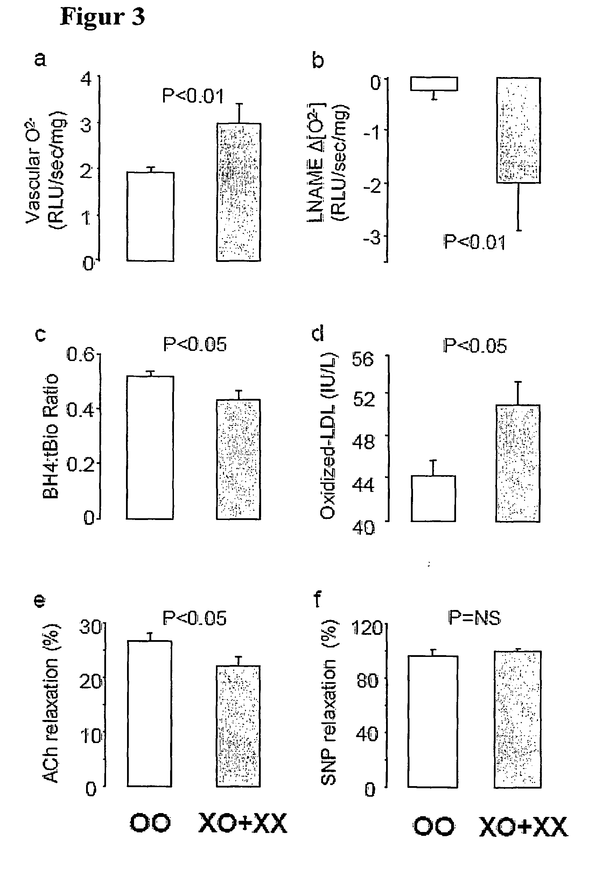

Figur 3: Das Auftreten des X-Haplotyps war mit einer höheren Gesamtsuperoxid (O2⁻) Produktion (a) und größerem L-NAME-inhibierbarem delta(O2⁻) (b) in menschlichen Saphenusvenen assoziiert. Der X-Haplotyp war auch mit einem signifikant niedrigerem Plasma BH4:tBio-Quotienten (c) und höheren Plasmakonzentrationen von oxidiertem LDL (ox-LDL, d) assoziiert. Weiterhin war der X-Haplotyp mit einer geringeren maximalen Vasorelaxation als Antwort auf Acetylcholin (ACh, e) in Saphenusvenen assoziiert. Der X-Haplotyp hat dagegen keinen Einfluss auf die vasomotorische Antwort auf Natrium-Nitroprussid (SNP, f).

**Tabelle 1: Demographische Charakteristika der Patienten**

| | |
|---|---|
| Zahl der Patienten | 347 |
| Männlich/weiblich | 292/55 |
| Alter (Jahre) | 65,42 ± 0,49 |
| Saphenusvenen (n) | 289 |
| Interne Arterien der Mamma (n) | 166 |
| | |
| **Risikofaktoren** | |
| Bluthochdruck (%) | 72,7 |
| Hypercholesterinämie (%) | 71,6 |
| Raucher (derzeitig/ehemalig) (%) | 8,6/69,8 |
| Diabetes Mellitus (%) | 27,5 |
| Familiäre Vorgeschichte koronarer Arterienerkrankungen (%) | 63,3 |
| Körpermassenindex (Kg/m²) | 28,04 ± 0,34 |
| Triglyceride (mmol/l) | 1,65 ± 0,09 |
| Cholesterin (mmol/l) | 4,07 ± 0,08 |
| Hochdichte Lipoproteine, HDL (mmol/l) | 1,10 ± 0,02 |
| C-reaktives Protein (mg/l) | 2,48 ± 0,07 |
| | |
| **Plasmabiopterinkonzentrationen** | |
| Plasma-Tetrahydrobiopterin (nmol/l)^{#} | 20,46[10,13-41,86] |
| Plasma-Dihydrobiopterin (mmol/l)^{#} | 15,19[11,77-19,41] |
| Plasma-Biopterin (mmol/l)^{#} | 4,09[2,83-6,98] |
| Plasma-Gesamt-Biopterin (mmol/l)^{#} | 46,11[30,04-71,33] |
| BH4/tBio-Quotient | 0,49 ± 0,018 |
| | |
| **Medikation (%)** | |
| Statin | 90,0 |
| AGEi/ARB | 66,0 |
| Kalziumkanalblocker | 40,8 |
| B-Blocker | 74,6 |
| Aspirin | 87,1 |

| | |
|---|---|
| ACEI: Inhibitoren des Angiotensin-konvertierenden Enzyms; ARB: Angiotensinrezeptorblocker; Werte ausgedrückt als Mittelwert ± Standardabweichung des Mittelwertes; ^{#}Werte ausgedrückt als Median (25.-75. Percentil). | |

### Beispiele

### Material und Methoden

### Probanden

347 Patienten mit CAD, die einer Koronaren Bypass-Operation (CABG) unterzogen wurden, wurden am John Radcliffe Hospital, Oxford, UK, untersucht. Jegliche entzündliche, infektive, Leber- oder Nierenerkrankung, bösartige Erkrankung, jeder akute koronare Zwischenfall während der letzten 2 Monate oder jedes klinisch offenkundige Herzversagen stellte ein Auschlusskriterium dar. Weiterhin wurden Patienten ausgeschlossen, die nicht steroidale antientzündliche Medikamente, oder die Nahrungsergänzungsmittel mit Folsäure oder antioxidativen Vitaminen einnahmen. Demographische Charakteristika der Patienten sind in Tabelle 1 gezeigt. Die Studie wurde durch das zuständige "Research Ethics Committee" bewilligt und jeder teilnehmende Patient gab eine schriftliche Einwilligungserklärung ab.

### Plasma und Gewebeproben

Blutproben wurden nach nächtlichem Fasten direkt vor der Operation entnommen. Die Proben wurden bei 2.500 Upm für 10 Minuten zentrifugiert und Serum oder Plasma wurde bei -80 °C bis zur Untersuchung aufbewahrt.

Proben von Saphenusvenen (SV) und der internen Arterie der Mamma (Arteria mammaria interna; IMA) wurden zur Zeit der CABG Operation entnommen, wie beschrieben (6, 20, 21). Gepaarte Gefäßsegmente wurden zur Messung des Biopteringehaltes in flüssigem Stickstoff eingefroren und bei -80 °C gelagert oder wurden für funktionelle Studien innerhalb von 30 Minuten in eiskaltem Krebs-Henseleit Puffer ins Labor verbracht (6, 20, 21).

### DNA Extraktion und Genotypisierung

Genomische DNA wurde mittels Standardmethoden aus 2 ml Gesamtblut extrahiert (QIAamp DNA blood Midi Kit, Qiagen, Germantown, Maryland). Der *GCH1* Haplotyp wurde mittels des validierten PyrosequencingTM Assays durch Typisierung von 3 *GCH1* Nukleotidpolymorphismen ("single nucleotide polymorphisms") (dbSNP rs8007267G>A, dbSNP rs3783641A>T und dbSNP rs10483639C>G) wie beschrieben (18, 19) diagnostiziert.

### RNA Isolierung und Echtzeit quantitative polymerase Kettenreaktion (RT qPCR)

In flüssigem Stickstoff gefrorene vaskuläre Ringe (IMA und SV) wurden zunächst mit Trizol-Reagenz lysiert und nachfolgend wurde RNA aus der wässrigen Phase unter Verwendung des RNeasy Micro Kit (Qiagen) gereinigt. RNA wurde in cDNA konvertiert (Superscript II reverse transcriptases, Invitrogen) und dann einer quantitativen PCR unter Verwendung des Taq-Man Systems (Applied Biosystems Assay ID GCH = Hs00609198_ml, Assay ID GAPDH = Hs02758991_gl) unterzogen und auf einem iCyclerIQ (Biorad) analysiert. Die relative Expression wurde unter Verwendung der 2^{-ΔΔC(T)} Methode berechnet, die in (22) beschrieben ist.

### Bestimmung der Biopterinkonzentrationen in Plasma und in der Gefäßwand

Konzentrationen von BH4, BH2 und Biopterin in Plasma- oder Gefäßgewebe-Lysaten wurden jeweils unabhängig von einander von der gleichen Probe bestimmt, und zwar durch HPLC gefolgt von serieller elektrochemischer und Fluoreszenz-Detektion, wie in (23) beschrieben. Gesamt-Biopterine (tBio) wurden durch Zusammenfassung von BH4, BH2 und B quantifiziert. Biopterinkonzentrationen wurden als pmol/g Gewebe für Gefäße und nmol/l für Plasma ausgedrückt.

### Bestimmung der vaskulären Superoxidproduktion

Die vaskuläre Superoxidproduktion wurden in gepaarten Segmenten von SV wie beschrieben (6, 24) unter Verwendung von Lucigenin-verbesserter Chemilumineszenz gemessen. Gefäße wurden logitudinal geöffnet, um die endoteliale Oberfläche zu exponieren und für 20 Minuten in oxigeniertem (95 % O₂/5 % CO₂ Krebs-HEPES Puffer (pH = 7,4) bei 37 °C äquilibriert. Lucigenin-verbesserte Chemilumineszenz wurde mittels gering konzentriertem Lucigenin (5 µmol/l) gemessen (21). Als Maß für "eNOS coupling" wurde die NOS-vermittelte Superoxidproduktion gemessen, die als Differenz in der Superoxidproduktion nach 20-minütiger Inkubation mit dem NOS-Inhibitor N^{G}-Nitro-L-argininmethylester (L-NAME; 100 µmol/l) abgeschätzt wurde.

### Vasomotorische Studien

Die Endothel-abhängige und Endotheli-unabhängige Dilatation wurde in zur Zeit des CABG gewonnenen SV mittels isometrischer Spannungsstudien bestimmt (6, 20, 21). Von jedem Gefäß wurden jeweils vier Ringe mit Phenylephrin (3 x 10⁻⁶ mol/l) prä-kontrahiert, dann wurde die Endothel-abhängige Relaxation unter Verwendung von Acetylcholin (ACh, 10⁻⁹ mol/l bis 10⁻⁵ mol/l) quantifiziert. Schließlich wurde, wie bereits beschrieben (6, 20, 21) die Relaxation als Reaktion auf den Endothel-unabhängigen NO-Donor Natriumnitroprussid (SNP, 10⁻¹⁰ mol/l bis 10⁻⁶ mol/l) in Gegenwart von L-NAME (100 µmol/l) bestimmt.

### Bestimmung und der Konzentrationen des oxidierten LDLs und des C-reaktiven Proteins

Serumkonzentrationen des oxidierten LDLs (ox-LDL) wurden durch Enzym-gebundenen Immunosorbent Assay (ELISA) unter Verwendung kommerziell erhältlicher Kits (Mercodia, Schweden) gemessen. Serumkonzentrationen des C-reaktiven Proteins (CRP) wurden durch Immunonephelometrie mittels einer hochsensitiven Methode gemessen (Dade Behring Marburg GmbH, Marburg, Deutschland).

### Statistische Analyse

Alle Variablen wurden mittels des Kolmokorov-Smirnov Tests auf normale Verteilung getestet. Normal verteilte Variablen werden als Mittelwert ± Standardabweichung des Mittelwertes dargestellt, während nicht normal verteilten Variablen zur Analyse logarithmisch transformiert wurden und als Median (25. bis 75. Percentil) zusammen mit der Spannweite angegeben werden. Der Vergleich zwischen der Grundlinie und den demographischen Charakteristika zwischen den drei Genotypen wurde unter Verwendung von "one-way ANOVA" für multiple Vergleiche durchgeführt, während ein ungepaarter t-Test zum Vergleich von Variablen zwischen zwei Gruppen (für rezessive Modelle) verwendet wurde.

Eine univariante Analyse wurde mittels Kalkulation des Pearson's Koeffizienten durchgeführt. Eine mulivariante Analyse wurde zur Untersuchung des Defekts des Genotyps auf das vaskuläre Biopterin bzw. Plasma-Biopterin, vaskuläres Superoxid, L-NAME-induzierte Veränderungen von vaskulärem Superoxid oder maximalen Relaxationen auf ACh als abhängige Variablen verwendet. Als unabhängige Variablen wurde *GCH1* Genotyp und die klinischen Charakteristika (Alter, Geschlecht, Diabetes Mellitus, Rauchen, Dyslipidämie, Bluthochdruck, Körpergewichtsindex und Medikamentenverabreichungen), die eine Assoziation mit der abhängigen Variablen in der univarianten Analyse bei einem Level von 15 % zeigten. Eine rückwärtig Eliminationsmethode wurden in allen Modellen angewendet, mit *P* > 0,1 als Schwellenwert zur Entfernung einer Variable aus dem Modell. Alle *P*-Werte wurden mit einem zweiseitigen Test ermittelt und *P* < 0,05 wurde als statistisch signifikant angesehen. Alle statistischen Analysen wurden mittels SPSS 12.0 durchgerührt.

### Ergebnisse

In 347 Patienten mit koronarer Herzerkrankung wurde der *GCH1* Haplotyp untersucht. Die Konkordanz der zur Definition des Haplotyps verwendeten drei SNPs betrug 100 %. Allel-Häufigkeiten des O und X Haplotyps betrugen 84,3 % und bzw. 15,7 %, wobei die Genotyp-Häufigkeit von OO 70,6 %, von XO 27,4 % und von XX 2,0 % betrugen. Dies stimmt mit der Hardy-Weinberg-Verteilung und in anderen Kohorten durchgeführten Studien (18, 19) überein.

Zunächst wurde die Wirkung des *GCH1* Haplotyps auf die Konzentration des Plasma-Biopterins untersucht. Patienten mit dem Haplotyp X zeigten signifikant geringere Plasmakonzentrationen, sowohl von BH4 als auch von Gesamt-Biopterin im Vergleich zu Patienten, die für den verbreiteten O Haplotyp homozygot sind. Der Median der Plasma-BH4-Konzentrationen in Patienten mit dem XX Genotyp war im Vergleich zu OO Patienten um ungefähr 80 % vermindert. Sowohl Plasma-BH4- als auch Gesamt-Biopterin-Konzentrationen waren in einer stark Allel-abhängigen Weise vom OO, XO und XX Genotyp reduziert (Figur 1).

Da es wahrscheinlich ist, dass vaskuläre Biopterin-Konzentrationen für die eNOS-Regulation im Endothel wichtiger sind, und diese möglicherweise anders reguliert werden als Plasma-Biopterin-Konzentrationen, wurde nachfolgend die Wirkung des *GCH1* Haplotyps auf vaskuläre Biopterin-Konzentrationen sowohl in SV als auch in IMA untersucht. Die Anwesenheit des X Haplotyps war mit signifikant niedrigeren Konzentrationen vaskulären BH4 und tBio sowohl in SV als auch IMA assoziiert (Figur 1). Diese Ergebnisse sprechen für einen direkten Effekt des *GCH1* Haplotyps auf die Biopterin-Synthese.

Um zu untersuchen, ob die Wirkung des *GCH1* Haplotyps auf die Biopterin-Konzentrationen durch Veränderungen in der *GCH1* Genexpression hervorgerufen wurden, wurden die *GCH1* mRNA in SV und IMA Proben von Patienten mit verschiedenen *GCH1* Genotypen mittels QRT-PCR quantifiziert. Es wurde beobachtet, dass der XX Genotyp mit einer signifikant verminderten vaskulären *GCH1* Expression im Vergleich zum OO Genotyp assoziiert war (Figur 2). Weiterhin waren vaskuläre *GCH1* mRNA Konzentrationen über alle Genotypen mit BH4-Konzentrationen sowohl im Plasma (r = 0,394, *P* = 0,010) als auch vaskulär (r = 0,336, *P* = 0,042) korreliert.

Weiterhin wurde untersucht, ob diese genetisch bestimmten Unterschiede in den Biopterin-Konzentrationen die NO-vermittelte endotheliale Funktion und vaskuläre Superoxidproduktion beeinflussen könnte. Da der homozygote X Haplotyp mit 2 % in der Bevölkerung selten ist, wurde ein rezessives Modell verwendet, bei dem Patienten mit und ohne X Haplotyp verglichen wurden. Patienten mit dem X Haplotyp (z.B. XO oder XX Genotyp) zeigten signifikant geringere BH4- und Gesamt-Biopterin-Konzentrationen als OO Patienten, sowohl in Plasma (16,6[5,2-28,3] und 41,2[23,4-54,9] vs 21,57[12,1-45,24] und 48,3[32,6-76,4] mnol/l, *P* < 0,05 für beide) als auch in SV (0,71[0,22-0,93] und 3,27[0,22-0,93] vs 1,10[0,57-1,78] und 4,3[2,4-7,6] nmol/l, *P* < 0,05 für beide).

In Trägern des X Haplotyps war die vaskuläre Superoxidproduktion signifikant erhöht (Figur 3). Um den spezifischen Beitrag des "eNOS coupling" zu untersuchen, wurden die Unterschiede in der vaskulären Superoxidproduktion nach NOS-Inhibierung durch L-NAME quantifiziert. Die Inkubation von Gefäßen mit L-NAME verminderte die Superoxidproduktion in einem signifikant größeren Maße in Trägem des X Haplotyps im Vergleich zu O-Homozygoten (Figur 3). Die Annahme eines erhöhten oxidativen Stresses in Trägem des X Haplotyps wurde auch unterstützt durch die Messung eines geringeren BH4:Gesamt-Biopterin-Quotienten und geringeren Konzentrationen oxidierten LDLs (Figur 3).

Um zu untersuchen, wie der *GCH1* Haplotyp und die damit assoziierten Unterschiede in Biopterin-Konzentrationen die NO-vermittelte endotheliale Funktion modulieren, wurde die vasomotorische Antwort von Gefäßsegmenten auf ACh in einem Organbad-System quantifiziert. Vasorelaxations-Antworten auf ACh waren in Trägem des X Haplotyps gegenüber O-Homozygoten signifikant reduziert, wohingegen Endothel-unabhängige vasomotorische Antworten auf den direkten NO-Donor, SNP, zwischen beiden Genotypen identisch waren (Figur 3).

### Multivariante Analyse

BH4-Korizentrationen und endotheliale Funktion könnte durch viele Faktoren verändert werden. Daher wurde ein multivarianter Analyseansatz gewählt, um das Verhältnis zwischen *GCH1* Haplotyp und Biopterin-Konzentrationen zu analysieren, wobei andere klinische demographische Faktoren einbezogen wurden. Der X Haplotyp stellte sich als unabhängiger Prädiktor sowohl für Plasma- als auch vaskuläre BH4-Konzentrationen heraus (Plasma: β(SE) -11,764 (4,722), *P* = 0,014; SV: β(SE) -0,306 (0,147), *P* = 0,039). Weiterhin war der X Haplotyp mit der vaskulären Superoxidproduktion unabhängig assoziiert (SV: β(SE) 1,196 (0,573), *P* = 0,04), als auch die maximale Relaxation in Reaktion auf ACh (β(SE) -5,304 (2,355), *P* = 0,026).

### Diskussion

Obwohl BH4 ein für den Erhalt eines funktionellen eNOS-Status im vaskulären Endothelium ein essenzieller Co-Faktor ist, ist über die pathophysiologische Kontrolle von endothelialen BH4-Konzentrationen im Menschen wenig bekannt. In experimentellen Tiermodellen sind artherosklerotische Risikofaktoren wie Diabetes Mellitus (16), Bluthochdruck (25) oder Dislipidämie (26) mit vaskulärer BH4-Defizienz assoziiert worden, ein Effekt, der in erster Linie durch intrazelluläre Oxidation von BH4 in BH2 und B mittels reak-tiver Sauerstoffspezies (wie Peroxinitrit) vermittelt wird (8, 27). Obwohl oxidativer Stress scheinbar ein wichtiger Regelungsmechanismus für vaskuläre BH4-Bioverfügbarkeit darstellt, bleiben die Mechanismen, die die Biosynthese von BH4 in vaskulären Erkrankungen kontrollieren, umstritten.

Die Biosynthese von BH4 beginnt mit GTPCH, die die Umsetzung von GTP in Dihydroneopterintriphosphat katalysiert, was dann durch 6-Pyruvoyltetrahydrobiopterinsyntase und Sepiapterinreduktase in BH4 umgewandelt wird (28). Im Gegensatz zu den letztgenannten Enzymen ist die Aktivität von GTPCH in den meisten Geweben geschwindigkeitsbestimmend (29) und wird oft als der Hauptregulator der BH4-Synthese angesehen. Das GTPCH-kodierende *GCH1* Gen wird in verschiedenen Zellarten wie Makrophagen (29), endothelialen Zellen (30) und anderen Zellen exprimiert. Experimentelle Studien legen nahe, dass die *GCH1* Expression in endothelialen Zellen oder Makrophagen durch verschieden Stimuli wie Insulin (31), Stress durch Scherkräfte (32) und Entzündungen (33, 34) induziert werden kann. Durch experimentelle Mausmodelle mit Veränderungen in der vaskulärspezifischen *GCH1* Expression (9, 15) konnte gezeigt werden, dass GTPCH ein Schlüsselregulator der vaskulären BH4-Konzentration *in vivo* darstellt. Trotz alledem war es bisher unklar, in wie fern eine veränderte *GCH1* Expression in menschlichen Gefäßwänden einen Einfluss auf vaskuläre BH4-Konzentrationen hat oder in wie fern die veränderte *GCH1* Expression Auswirkungen auf die endotheliale Funktion in Patienten mit vaskulären Erkrankungen hat.

### Referenzen

1. Harrison D, Griendling KK, Landmesser U, Hornig B, Drexler H. Role of oxidative stress in atherosclerosis. Am J Cardiol 2003;91:7A-11A.
2. Channon KM. Tetrahydrobiopterin: regulator of endothelial nitric oxide synthase in vascular disease. Trends Cardiovasc Med 2004;14:323-7.
3. Stroes E, Kastelein J, Cosentino F, et al. Tetrahydrobiopterin restores endothelial function in hypercholesterolemia. J Clin Invest 1997;99:41-6.
4. Heitzer T, Brockhoff C, Mayer B, et al. Tetrahydrobiopterin improves endotheliumdependent vasodilation in chronic smokers : evidence for a dysfunctional nitric oxide synthase. Circ Res 2000;86:E36-E41.
5. Antoniades C, Shirodaria C, Warrick N, et al. 5-methyl-tetrahydrofolate rapidly improves endothelial function and decreases superoxide production in human vessels: effects on vascular tetrahydrobiopterin availability and eNOS coupling. Circulation 2006;114:1193-1201.
6. Shirodaria C, Antoniades C, Lee J, et al. Global improvement of vascular function and redox state with low-dose folic acid: implications for folate therapy in patients with coronary artery disease. Circulation 2007; 115:2262-70.
7. Kuzkaya N, Weissmann N, Harrison DG, Dikalov S. Interactions of peroxynitrite, tetrahydrobiopterin, ascorbic acid, and thiols: implications for uncoupling endothelial nitric-oxide synthase. J Biol Chem 2003;278:22546-54.
8. Stroes ES, van Faassen EE, Yo M, et al. Folic acid reverts dysfunction of endothelial nitric oxide synthase. Circ Res 2000;86:1129-34.
9. Verhaar MC, Wever RM, Kastelein JJ, van Dam T, Koomans HA, Rabelink TJ. 5-methyltetrahydrofolate, the active form of folic acid, restores endothelial function in familial hypercholesterolemia. Circulation 1998;97:237-41.
10. Khoo JP, Nicoli T, Alp NJ, Fullerton J, Flint J, Channon KM. Congenic mapping and genotyping of the tetrahydrobiopterin-deficient hph-1 mouse. Mol Genet Metab 2004;82:251-4.
11. Meininger CJ, Marinos RS, Hatakeyama K, et al. Impaired nitric oxide production in coronary endothelial cells of the spontaneously diabetic BB rat is due to tetrahydrobiopterin deficiency. Biochem J 2000;349:353-356.
12. Landmesser U, Dikalov S, Price SR, et al. Oxidation of tetrahydrobiopterin leads to uncoupling of endothelial cell nitric oxide synthase in hypertension. J Clin Invest 2003;111:1201-9.
13. Huang A, Zhang YY, Chen K, Hatakeyama K, Keaney JF, Jr. Cytokine-stimulated GTP cyclohydrolase I expression in endothelial cells requires coordinated activation of nuelear factor-kappaB and Statl/Stat3. Circ Res 2005;96:164-71.
14. Alp NJ, McAteer MA, Khoo J, Choudhury RP, Channon KM. Increased endothelial tetrahydrobiopterin synthesis by targeted transgenic GTP-cyclohydrolase I overexpression reduces endothelial dysfunction and atherosclerosis in ApoE-knockout mice. Arterioscler Thromb Vasc Biol 2004;24:445-450.
15. Cosentino F, Barker JE, Brand MP, et al. Reactive oxygen species mediate endothelium-dependent relaxations in tetrahydrobiopterin-deficient mice. Arterioscler Thromb Vasc Biol 2001;21:496-502.
16. Khoo JP, Zhao L, Alp NJ, et al. A pivotal role for tetrahydrobiopterin in pulmonary hypertension. Circulation 2005;111:2126-2133.
17. Alp NJ, Mussa S, Khoo J, et al. Tetrahydrobiopterin-dependent preservation of nitric oxide-mediated endothelial function in diabetes by targeted transgenic GTP-cyclohydrolase I overexpression. J Clin Invest 2003;112:725-735.
18. Tegeder I, Costigan M, Griffin RS, et al. GTP cyclohydrolase and tetrahydrobiopterin regulate pain sensitivity and persistence. Nat Med 2006;12:1269-77.
19. Lotsch J, Belfer I, Kirchhof A, et al. Reliable Screening for a Pain-Protective Haplotype in the GTP Cyclohydrolase 1 Gene (GCH1) Through the Use of 3 or Fewer Single Nucleotide Polymorphisms. Clin Chem 2007;53:1010-5.
20. Guzik TJ, Mussa S, Gastaldi D, et al. Mechanisms of increased vascular superoxide production in human diabetes mellitus: Role of NAD(P)H oxidase and endothelial nitric oxide synthase. Circulation 2002;105:1656-1662.
21. Tegeder I, Costigan M, Griffin RS, et al. GTP cyclohydrolase and tetrahydrobiopterin regulate pain sensitivity and persistence. Nat Med 2006;12:1269-1277.
22. Shi L, Norling LA, Lau AS, Krejci S, Laney AJ, Xu Y. Real time quantitative PCR as a method to evaluate simian virus 40 removal during pharmaceutical protein purification. Biologicals 1999;27:253-62.
23. Heales S, Hyland K. Determination of quinonoid dihydrobiopterin by highperformance liquid chromatography and electrochemical detection. J Chromatogr 1989;494:77-85.
24. Skatchkov MP, Sperling D, Hink U, et al. Validation of lucigenin as a chemiluminescent probe to monitor vascular superoxide as well as basal vascular nitric oxide production. Biochem Biophys Res Commun 1999;254:319-24.
25. Guzik TJ, West NEJ, Black E, et al. Vascular superoxide production by NAD(P)H oxidase: association with endothelial dysfunction and clinical risk factors. Circ Res 2000;86:e85-e90.
26. Channon KM, Guzik TJ. Mechanisms of superoxide production in human blood vessels: relationship to endothelial dysfunction, clinical and genetic risk factors. J Physiol Pharmacol 2002;53:515-24.
27. Lewontin RC. The Interaction of Selection and Linkage. Ii. Optimum Models. Genetics 1964;50:757-82.
28. Gaut BS, Long AD. The lowdown on linkage disequilibrium. Plant Cell 2003;15:1502-6.
29. Barrett JC, Fry B, Maller J, Daly MJ. Haploview: analysis and visualization of LD and haplotype maps. Bioinformatics 2005;21:263-5.
30. Hardy GH. Mendelian proportions in a mixed population. Science 1908;28:49-50.
31. Thum T, Fraccarollo D, Schultheiss M, et al. Endothelial nitric oxide synthase uncoupling impairs endothelial progenitor cell mobilization and function in diabetes. Diabetes 2007;56:666-74.
32. Laursen JB, Somers M, Kurz S, et al. Endothelial regulation of vasomotion in apoEdeficient mice: implications for interactions between peroxynitrite and tetrahydrobiopterin. Circulation 2001;103:1282-8.
33. Shinozaki K, Nishio Y, Okamura T, et al. Oral administration of tetrahydrobiopterin prevents endothelial dysfunction and vascular oxidative stress in the aortas of insulinresistant rats. Circ Res 2000;87:566-573.
34. Shinozaki K, Hirayama A, Nishio Y, et al. Coronary endothelial dysfunction in the insulin-resistant state is linked to abnormal pteridine metabolism and vascular oxidative stress. J Am Coll Cardiol 2001;38:1821-8.
35. Werner-Felinayer G, Werner ER, Fuchs D, Hausen A, Reibnegger G, Wachter H. Tetrahydrobiopterin-dependent formation of nitrite and nitrate in murine fibroblasts. J Experim Med 1990;172:1599-1607.
36. Thony B, Auerbach G, Blau N. Tetrahydrobiopterin biosynthesis, regeneration and functions. Biochem J 2000;347 Pt 1:1-16.
37. Werner ER, Werner-Felmayer G, Fuchs D, et al. Tetrahydrobiopterin biosynthetic activities in human macrophages, fibroblasts, THP-1, and T 24 cells. GTP-cyclohydrolase I is stimulated by interferon-gamma, and 6-pyruvoyl tetrahydropterin synthase and sepiapterin reductase are constitutively present. J Biol Chem 1990,265:3189-92.
38. Linscheid P, Schaffner A, Blau N, Schoedon G. Regulation of 6-pyruvoyltetrahydropterin synthase activity and messenger RNA abundance in human vascular endothelial cells. Circulation 1998;98:1703-6.
39. Antoniades C, Shirodaria C, Crabtree M, et al. Altered Plasma vs. Vascular Biopterins in Human Atherosclerosis Reveal Relationships Between Endothelial Nitric Oxide Synthase Coupling, Endothelial Function and Inflammation. Circulation 2008;(In press).
40. Zhang L, Rao F, Zhang K, et al. Discovery of common human genetic variants of GTP cyclohydrolase 1 (GCH1) governing nitric oxide, autonomic activity, and cardiovascular risk. J Clin Invest 2007;117:2658-71.
41. Vasquez-Vivar J, Kalyanaraman B, Martasek P, et al. Superoxide generation by endothelial nitric oxide synthase: the influence of cofactors. Proc Natl Acad Sci U S A 1998;95:9220-5.
42. Kwok, Pharmacogenomics 1, 95, (2000)

## Patentansprüche

1. Verfahren zur Diagnose einer genetischen Prädisposition für eine Gefäßerkrankung, insbesondere für eine koronare Arterienerkrankung (CAD), aufweisend
- Bereitstellen einer Probe, enthaltend eine von dem Gen *GCH1* abgeleitete Nukleinsäure; und
- Bestimmen der An- oder Abwesenheit eines Nukleotidpolymorphismus (SNP) des Gens *GCH1* in der Nukleinsäure, wobei der SNP ausgewählt ist aus der Gruppe bestehend aus rs8007267 G>A, rs3783641 A>T und rs10483639 C>G,
wobei die Anwesenheit mindestens eines der genannten SNPs eine genetische Prädisposition für eine Gefäßerkrankung anzeigt.

2. Verfahren nach Anspruch 1, wobei die Nukleinsäure genomische DNS, unprozessierte RNS (hnRNS) oder eine davon abgeleitete cDNS ist.

3. Verfahren nach Anspruch 1 oder 2, wobei der Nachweis der An- oder Abwesenheit eines SNP des Gens *GCH1* in der Nukleinsäure durch Sequenzierung, Hybridisierung, Restriktions-Fragment-Analyse, Oligonukleotidligation oder allel-spezifische PCR erfolgt.

4. Verfahren nach Anspruch 1 bis 3, wobei die bereitgestellte Probe aus einer Körperflüssigkeit gewonnen wird.

5. Verfahren nach Anspruch 1 bis 3, wobei die bereitgestellte Probe aus einem Gewebe gewonnen wird.

6. Verwendung eines Verfahrens nach Anspruch 1 bis 5 zur Diagnose einer genetischen Prädisposition für eine Gefäßerkrankung in einem Individuum.

7. Verwendung eines Kits zur Diagnose einer genetischen Prädisposition für eine Gefäßerkrankung in einem Individuum, insbesondere einer koronaren Arterienerkrankung (CAD), aufweisend mindestens eine Sonde und/oder einen Primer zum Nachweis eines SNP des Gens *GCH1*, wobei das SNP ausgewählt ist aus der Gruppe bestehend aus rs8007267 G>A, rs3783641 A>T und rs10483639 C>G.

8. Verwendung nach Anspruch 7, wobei der Primer ein Primer für eine Amplifikationsreaktion oder ein Primer für eine Sequenzierungsreaktion ist.

9. Verwendung nach Anspruch 8, wobei der Primer für eine Amplifikationsreaktion ein Primer nach SEQ ID No. 1 bis 6 oder ein Primer für eine Sequenzierungsreaktion nach SEQ ID No. 7 bis 9 ist.

## Claims

1. Method for diagnosing a genetic predisposition for a vascular disease, in particular for a coronary artery disease (CAD), comprising
- providing a sample containing a nucleic acid derived from the gene *GCH1*; and
- determining the presence or absence of a nucleotide polymorphism (SNP) of the gene *GCH1* in the nucleic acid, wherein the SNP is selected from the group consisting of rs8007267 G>A, rs3783641 A>T, and rs10483639 C>G,
wherein the presence of at least one of said SNPs indicates a genetic predisposition for a vascular disease.

2. The method according to claim 1, wherein the nucleic acid is a genomic DNA, unprocessed RNA (hnRNA) or a cDNA derived therefrom.

3. The method according to claim 1 or 2, wherein the detection of the presence or absence of an SNP of the gene *GCH1* in the nucleic acid is carried out by sequencing, hybridization, restriction-fragment-analysis, oligonucleotide ligation or allele-specific PCR.

4. The method according to any of claims 1 to 3, wherein the sample as provided is obtained from a body fluid.

5. The method according to any of claims 1 to 3, wherein the sample as provided is obtained from a tissue.

6. Use of the method according to any of claims 1 to 5 for diagnosing a genetic predisposition for a vascular disease in an individual.

7. Use of a kit for diagnosing a genetic predisposition for a vascular disease in an individual, in particular for a coronary artery disease (CAD), comprising at least one probe and/or one primer for a detection of an SNP of the gene *GCH1*, wherein the SNP is selected from the group consisting of rs8007267 G>A, rs3783641 A>T, and rs10483639 C>G.

8. The use according claim 7, wherein the primer is a primer for an amplification reaction or a primer for a sequencing reaction.

9. The use according claim 8, wherein the primer for an amplification reaction is a primer according to SEQ ID No. 1 to 6, or is a primer for a sequencing reaction according to SEQ ID No. 7 to 9.

## Revendications

1. Procédé pour le diagnostic d'une prédisposition génétique à une maladie vasculaire, en particulier une maladie des artères coronaires (CAD), présentant
- la préparation d'un échantillon contenant un acide nucléique dérivé du gène GCHI ; et
- la détermination de la présence ou de l'absence d'un polymorphisme nucléotidique (SNP) du gène GCHI dans l'acide nucléique, le SNP étant choisi dans le groupe constitué par rs8007267 G>A, rs3783641 A>T et rs10483639C>G,
la présence d'au moins l'un des SNP cités indiquant une prédisposition génétique pour une maladie vasculaire.

2. Procédé selon la revendication 1, l'acide nucléique étant de l'AND génomique, de l'ARN non converti (hnARN) ou un cADN qui en est dérivé.

3. Procédé selon les revendications 1 ou 2, l'identification de la présence ou de l'absence d'un SNP du gène GCHI dans l'acide nucléique étant effectuée par séquençage, hybridation, analyse de fragments de restriction, ligation d'oligonucléotides ou amplification en chaine par polymérase allèle spécifique.

4. Procédé selon les revendications de 1 à 3, l'échantillon préparé provenant d'un liquide corporel.

5. Procédé selon les revendications de 1 à 3, l'échantillon préparé provenant d'un tissu.

6. Utilisation d'un procédé selon les revendications de 1 à 5 pour le diagnostic d'une prédisposition génétique à une maladie vasculaire chez un individu.

7. Utilisation d'un kit pour le diagnostic d'une prédisposition génétique à une maladie vasculaire chez un individu, en particulier une prédisposition à une maladie des artères coronariennes (CAD), présentant au moins une sonde et/ou une amorce pour l'identification d'un SNP d'un gène GCHI, le SNP étant choisi dans le groupe constitué par rs8007267 G>A, rs3783641 A>T et rs10483639C>G.

8. Utilisation selon la revendication 7, l'amorce étant une amorce pour une réaction d'amplification ou une amorce pour une réaction de séquençage.

9. Utilisation selon la revendication 8, l'amorce pour une réaction d'amplification étant une amorce selon SEQ ID N°1 à 6 ou une amorce pour une réaction de séquençage selon SEQ ID N°7 à 9.
